# FASCICULE DE BREVET EUROPEEN

(11) **EP 2 760 486 B1**
(45) Date de publication et mention de la délivrance du brevet: **29.05.2024**
(21) Numéro de dépôt: 12766443.1
(22) Date de dépôt: 26.09.2012
(51) Int. Cl.: A61L 9/20, F24F 8/22

(54) **PROCÉDÉ ET DISPOSITIF DE PURIFICATION ET DE DÉSODORISATION DE L'AIR**
VERFAHREN UND VORRICHTUNG ZUR REINIGUNG UND DESODORISIERUNG VON LUFT
METHOD AND DEVICE FOR PURIFICATION AND DEODORIZATION OF AIR

(30) Priorité: 27.09.2011 FR 1158657
(43) Date de publication de la demande: 06.08.2014
(73) Titulaire: TREFFLER INVENTIONS, 38000 Grenoble (FR)
(72) Inventeur: FAURIE, Jean-Michel, F-69380 Dommartin (FR); CHAVANON, Didier, F-38540 St Just Chaleyssin (FR)
(74) Mandataire: Lavoix
(86) Numéro de dépôt international: PCT/EP2012/069005
(87) Numéro de publication internationale: WO 2013/045515

(56) Documents cités:
- FR-A1- 2 892 951
- JP-A- 2005 342 509
- US-A1- 2003 113 246
- US-B1- 6 797 127
- US-B2- 6 589 489

## Description

La présente invention concerne un procédé de purification et/ou de désodorisation de l'air. L'invention concerne également un dispositif permettant de mettre en oeuvre ce procédé.

L'amélioration de la qualité de l'air devient une préoccupation majeure des autorités sanitaires. Elle peut notamment passer par une purification de l'air.

De nombreux procédés ont été décrits pour purifier l'air.

On connait, notamment de WO02/47799, un dispositif de purification de l'air comprenant une source de rayonnement UV et un photocatalyseur. On connaît également de WO2010/130965 un dispositif de purification d'air comprenant un média filtrant comprenant une cellule de photocatalyse présentant un filtre photocatalytique destiné à être irradié par une source lumineuse (254 nm) pour piéger des polluants chimiques et/ou microbiologiques. On connaît de WO2008/122871 la combinaison d'un traitement photocatalytique et le piégeage de particules pour la purification de l'air. L'utilisation d'une source lumineuse émettant des rayons de 250 à 270 nm est décrite comme présentant des effets germicides. On connaît enfin de WO2004/081458 un dispositif de filtration d'air comprenant un agent d'adsorption (charbon actif), un agent photocatalyseur (TiOs) et des moyens d'éclairement de l'agent photocatalyseur, ces éléments étant contenus dans deux modules distincts.

Cependant, les procédés et dispositifs déjà connus ne permettent pas de purifier d'une façon suffisamment efficace l'air. Une étude récente (Env. Risque Santé, 2011, vol.10, n°1, 35-45) a mis en évidence que les procédés de l'état de la technique génèrent, par dégradation des polluants initialement présents dans l'air, d'autres polluants qui ne sont pas dégradés. Certains dispositifs de l'état de la technique émettent également de l'ozone et/ou des NOx.

Il est également connu, notamment d'US6589489 et de JP2005342509, des procédés pour le traitement de l'air et installations pour la mise en oeuvre de ces procédés, notamment pour la purification de l'air, comprenant notamment la dégradation par l'ozone des polluants contenus dans l'air. L'ozone étant produit au moyen d'une lampe émettant notamment des rayonnements à 185 nm. Les lampes mises en oeuvre dans ces procédés sont des lampes émettant des rayonnements de 185 à 350 nm. Les lampes sont utilisées dans tous les étages du système de traitement d'air, l'ozone étant, par conséquent, produit dans tous les étages de l'installation. La quantité d'ozone générée est donc importante et le risque de libération de l'ozone en dehors du dispositif est également important voire systématique. Pour limiter la quantité d'ozone produit et les risques de libération de l'ozone à l'extérieur du dispositif, il est alors nécessaire de mettre en oeuvre, dans les dispositifs connus, des systèmes complexes, par exemple des systèmes catalytiques permettant d'adsorber l'ozone ou des filtres permettant de retenir sur certaines parties du dispositif les rayonnements à 185 nm limitant ainsi la production d'ozone.

Une diminution de la puissance de la lampe aurait pour conséquence la diminution de la puissance des autres rayonnements, notamment des rayonnements à 254 nm qui permettent, par photocatalyse, de décomposer les polluants compris dans l'air à traiter. Il en résulterait une dépollution moins efficace.

Il y a donc un intérêt à fournir un procédé de purification d'air amélioré par rapport aux procédés connus de l'état de la technique.

Un objectif de la présente invention est de fournir un procédé de purification de l'air qui soit efficace, industrialisable et économique, ne nécessitant pas de mise en oeuvre complexe pour l'élimination de l'ozone.

Un objectif de la présente invention est encore de fournir un procédé de purification de l'air produisant une quantité contrôlée d'ozone permettant de décomposer les polluants de l'air.

Un autre objectif de la présente invention est de fournir un procédé de purification et/ou de désodorisation de l'air qui ne génère pas de polluants supplémentaires par rapport aux polluants initialement contenus dans l'air.

Un autre objectif de la présente invention est de fournir un procédé qui puisse être utilisé dans des applications domestiques ou grand public, dans des applications industrielles, dans des salles blanches, dans les milieux hospitaliers, dans les bureaux, etc.

Un autre objectif de l'invention est de fournir un dispositif d'encombrement minimal et permettant de mettre en oeuvre ce procédé.

D'autres objectifs encore apparaîtront à la lecture de la description de l'invention qui suit.

Ces objectifs sont remplis par la présente invention qui concerne un procédé de purification et/ou de désodorisation d'air comprenant des polluants, tel que défini aux revendications.

De préférence, le ratio pour la monosource, quantité d'énergie des rayons à 185 nm/quantité d'énergie des rayons à 254 nm va de 0,005 à 0,05, pour une concentration en polluant allant de 1 à 5000 ppm, de préférence de 1 à 100 ppm, de manière plus préférée de 1 ppb à 10 ppm.

Comme expliqué plus loin, ces ratios sont obtenus en mettant en oeuvre une monosource lumineuse constituée de deux quartz l'un émettant des rayons à 185 nm et à 254 nm et l'autre émettant des rayons uniquement à 254 nm.

Le procédé selon l'invention peut en outre comprendre une dégradation des polluants contenus dans l'air par l'action de rayons UVA.

Les rayons UVA peuvent provenir d'une source lumineuse distincte ou provenir de la monosource émettant alors des rayons à 185 nm, à 254 nm et des rayons UVA, la monosource présente alors un ratio quantité d'énergie des rayons à 185 nm/quantité d'énergie des rayons à 254 nm et rayons UVA) allant de 0,005 à 0,05. De préférence, la monosource présente alors un ratio quantité d'énergie des rayons à 185 nm/quantité d'énergie des rayons à 254 nm et rayons UVA) allant de 0,005 à 0,05 pour une concentration en polluant allant de 1 à 5000 ppm, de préférence de 1 à 100 ppm de préférence de 1 à 100 ppm, de manière plus préférée de 1 ppb à 10 ppm.

Les sources lumineuses sont notamment des diodes, des diodes électroluminescentes (DEL) ou des lampes.

Par rayon à 254 nm on entend tous rayons ayant une longueur d'onde proche de 254 nm, notamment des rayons ayant une longueur d'onde comprise entre 250 et 260 nm. De manière plus particulière, par rayons à 254 nm, la présente invention entend également couvrir les rayons à 253,7 nm habituellement obtenus par utilisation de sources lumineuses standard à 254 nm.

Les rayons UVA sont notamment des rayons à 365 nm. Par rayons à 365 nm, on entend tous rayons ayant une longueur d'onde proche de 365 nm, notamment des rayons ayant une longueur d'onde comprise entre 350 et 375 nm.

Dans le cadre de la présente invention, on entend par purification d'air le traitement de l'air pour dégrader les polluants pouvant être contenus dans cet air. On entend également par purification d'air la stérilisation de l'air visant la dégradation des bactéries et virus pouvant être contenus dans cet air.

Dans le cadre de l'invention on entend par désodorisation de l'air le traitement de l'air pour supprimer les odeurs, par exemples les odeurs alimentaires, les odeurs de rejets industriels, les odeurs de solvants, etc.

Dans le cadre de la présente invention on entend par polluants tous produits chimiques, physiques et/ou biologiques pouvant être contenus dans l'air et qu'il convient de supprimer. Ces polluants peuvent être des produits néfastes pour l'environnement, pour la santé humaine ou animale ; ils peuvent également être des produits néfastes ou nocifs dans le cadre d'activités particulières, par exemple dans les salles d'hôpital qui doivent présenter des seuils de polluants, notamment bactéries, très faibles voire nulles, dans les salles blanches, mais également dans les chambres froides (notamment pour la conservation des aliments), les salles d'élevage, les pièces d'une habitation.

De préférence, les polluants chimiques sont notamment les allergènes, le formaldéhyde ou un polluant choisi dans le groupe des composés organiques volatils (COV) tel que par exemple l'éthylène, l'acétone, le toluène, l'acétaldéhyde, le xylène et l'éthane.

De préférence, les polluants biologiques sont les bactéries, les champignons et les virus.

De façon avantageuse, les rayons de longueur d'onde 254 nm permettent notamment une dégradation des polluants biologiques, notamment de type virus et bactéries.

Le procédé comprend donc une étape de dégradation totale ou partielle des polluants de l'air par ozonolyse. Dans ce cas particulier, l'ozone provient de la réaction de l'oxygène contenu dans l'air avec les rayons de longueur d'onde 185 nm.

De façon avantageuse, l'ozonolyse permet une dégradation plus importante des polluants de l'air.

De manière particulièrement avantageuse, dans le procédé de l'invention, le contrôle de la quantité d'énergie correspondant au rayonnement de longueur d'onde 185 nm permet de contrôler la quantité d'ozone formé afin de ne pas relarguer ou de ne relarguer que très peu d'ozone à l'issue du procédé de purification de l'invention.

De façon avantageuse, la quantité d'énergie correspondant au rayonnement 185 nm est contrôlé de sorte que le procédé ne relargue pas d'ozone, l'ozone produit par réaction de l'oxygène avec les rayonnements 185 nm étant totalement consommé pour la dégradation des polluants et pour la réaction de photocatalyse.

Pour ce faire, la monosource lumineuse émettant des rayons à 185 nm et à 254 nm est constituée de deux quartz, un premier quartz émettant des rayons à 254 nm et à 185 nm et le cas échéant des rayons UVA (dans le cas d'une monosource émettant des rayons à 185 nm, à 254 nm et des rayons UVA) et un second quartz émettant des rayons à 254 nm ou le cas échéant des rayons à 254 nm et des rayons UVA. Le quartz émettant des rayons à 254 nm et à 185 nm et le cas échéant UVA présente une longueur et une surface inférieure à celui émettant uniquement les rayons à 254 nm ou le cas échéant uniquement les rayons à 254 nm et les rayons UVA. La monosource est une lampe bi-quartz 254 et 185 nm pour laquelle la longueur et la surface du quartz émettant des rayons à 185 nm et à 254 nm est réduite par rapport à la longueur du quartz n'émettant des rayons qu'à 254 nm. Cette longueur de quartz émettant des rayons à 185 nm et à 254 nm et éventuellement des UVA est ajustée ou adaptée notamment par rapport au débit de l'air passant et aux types de polluants à éliminer.

De manière générale, la monosource lumineuse ainsi que toutes les autres sources décrites dans la présente invention peuvent également émettre des rayons dans le visible.

De préférence, la quantité d'énergie correspondant au rayonnement de longueur d'onde 185 nm va de 0,05 à 100 W, de préférence de 0,1 à 100 W, de préférence de 0,1 à 15 W, de préférence de 0,5 à 15 W pour une concentration en polluant allant de 1 à 5000 ppm, de préférence de 1 ppb à 100 ppm.

De préférence, la quantité d'énergie correspondant au rayonnement 185 nm va de 1 à 100 W, de préférence de 1 à 25 W, de préférence de 0,05 à 25 W, pour un débit d'air entrant allant de 1 à 5000 m³/h, de préférence de 1 à 1000 m³/h.

Le procédé de la présente invention peut en outre comprendre une ou plusieurs des étapes suivantes, suivant le degré de pureté de l'air qu'il est envisagé d'obtenir :
- la dégradation des polluants par action de rayons UV, notamment rayons UVC; et/ou
- la dégradation des polluants par action de rayons UV, notamment rayons UVA; et/ou
- la dégradation des polluants par une autre étape de photocatalyse ; et/ou
- le piégeage des polluants par adsorption sur un support d'adsorption ;
chacune de ces étapes pouvant être mise en oeuvre plusieurs fois indépendamment les unes des autres et pouvant être combinées.

Ainsi et de préférence, le procédé selon l'invention peut en outre comprendre, suivant le degré de pureté de l'air qu'il est envisagé d'obtenir :
- la dégradation des polluants par action de rayons UV, notamment rayons UVC; ou
- la dégradation des polluants par action de rayons UV, notamment rayons UVA ; ou
- la dégradation des polluants par une autre étape de photocatalyse ; ou
- le piégeage des polluants par adsorption sur un support d'adsorption ; ou
- la dégradation des polluants par action de rayons UVC et par action de rayons UVA; ou
- la dégradation des polluants par action de rayons UVC, par action de rayons UVA, et la dégradation des polluants par une autre étape de photocatalyse ; ou
- la dégradation des polluants par action de rayons UVC, par action de rayons UVA, et le piégeage des polluants par adsorption sur un support d'adsorption ; ou
- la dégradation des polluants par action de rayons UVC, par action de rayons UVA, la dégradation des polluants par une autre étape de photocatalyse et le piégeage des polluants par adsorption sur un support d'adsorption ; ou
- la dégradation des polluants par action de rayons UVC et la dégradation des polluants par une autre étape de photocatalyse ; ou
- la dégradation des polluants par action de rayons UVC, la dégradation des polluants par une autre étape de photocatalyse et le piégeage des polluants par adsorption sur un support d'adsorption ; ou
- la dégradation des polluants par action de rayons UVC et le piégeage des polluants par adsorption sur un support d'adsorption ; ou
- la dégradation des polluants par action de rayons UVA, et la dégradation des polluants par une autre étape de photocatalyse ; ou
- la dégradation des polluants par action de rayons UVA, , la dégradation des polluants par une autre étape de photocatalyse et le piégeage des polluants par adsorption sur un support d'adsorption ; ou
- la dégradation des par action de rayons UVA, et le piégeage des polluants par adsorption sur un support d'adsorption ; ou
- la dégradation des polluants par une autre étape de photocatalyse et le piégeage des polluants par adsorption sur un support d'adsorption.

De manière connue, les rayons UVC sont notamment des rayons à 254 nm et les rayons UVA sont notamment des rayons à 365 nm.

Il doit être compris que dans les procédés de la présente invention les rayons UVC et UVA agissant dans les étapes complémentaires peuvent provenir d'au moins une autre source lumineuse ou de tout ou partie du quartz de la monosource émettant des rayons à 254 nm et le cas échéant des rayons UVA. La monosource émettant des rayons à 185 nm, 254 nm et des rayons UVA est alors telle que caractérisée ci-dessus.

Ainsi, la présente invention entend couvrir les combinaisons d'étapes menées avec la monosource seule par l'action des différents rayons qu'elle émet ainsi que des combinaisons d'étapes menées avec la monosource et d'autres sources lumineuses.

La photocatalyse est généralement mise en oeuvre par action de rayon UV en présence d'un catalyseur. La photocatalyse nécessite donc la présence d'au moins une source lumineuse. De préférence, les rayons UV sont des rayons UVC et/ou UVA, de préférence des rayons à 254 nm et/ou à 365 nm, par exemple des rayons de longueur d'onde 254 nm.

Le catalyseur est choisi parmi les photocatalyseurs supportés ou non supportés, notamment parmi les semi-conducteurs. Par exemple, le catalyseur comprend du titane ou du tungstène. De préférence, le catalyseur est le dioxyde de titane supporté ou non supporté. Le support peut notamment être du papier ; de la silice ou des composés comprenant de la silice, par exemple le quartz ; des tissus, notamment tissus de verre, par exemple tissus de quartz et silice ; des fibres de verre ; des tissus de fibres de verre ; des tissus de fibres optiques ; des céramiques ; ou un assemblage de ces supports. De manière préférée, le support est un assemblage de tissus de verre et de papier.

Le dépôt du catalyseur sur le support peut se faire de toute manière connue de l'homme du métier. Il peut notamment être effectué par pulvérisation du catalyseur sur le support ou par trempage du support dans une solution de catalyseur ou encore par magnétron pulsé haute puissance, ou encore par déposition de couches d'atomes. De préférence, le dépôt du catalyseur est effectué par pulvérisation du catalyseur sur le support ou par trempage du support dans une solution de catalyseur ou encore par magnétron pulsé haute puissance.

La mise en oeuvre ou non de l'étape de piégeage des polluants peut être déterminée par l'homme du métier notamment en fonction des polluants contenus dans l'air, de leur degré de dégradation, notamment s'ils produisent des sous-produits indésirables ou nocifs lors de leurs dégradations, du taux de purification souhaité, du type d'application envisagée. Par exemple, pour une application industrielle, le piégeage des polluants résiduels par un support d'adsorption ne sera pas nécessairement utile alors qu'il le sera dans des applications demandant un traitement plus poussé de l'air, par exemple dans des applications médicales. De façon avantageuse, le support d'adsorption permet de piéger l'ozone résiduel qui n'aurait pas été consommé lors de la dégradation des polluants et ainsi de relarguer un air dépourvu ou quasiment dépourvu d'ozone. Le support d'adsorption permet également de piéger les polluants qui n'auraient pas été dégradés ou pas complètement dégradés. Ainsi et de façon avantageuse, l'étape de piégeage permet un traitement fin de l'air et ainsi d'améliorer la qualité de l'air en sortie et donc d'obtenir un air très dépollué.

Le support d'adsorption peut être choisi parmi tous matériaux poreux susceptibles d'adsorber des polluants et éventuellement l'ozone et présentant un fort pouvoir adsorbant. Le support d'adsorption peut notamment être choisi parmi les charbons actifs ; les zéolithes ; les xerogels, notamment les xérogels à base de silice ; ou leurs mélanges.

De façon avantageuse, le support d'adsorption est régénéré par l'ozone résiduel, éventuellement présent, qui va venir dégrader les polluants qui se sont adsorbés à sa surface.

Dans un premier mode de réalisation particulier, le procédé (P1) selon l'invention comprend les étapes suivantes :
1) la dégradation des polluants par ozonolyse, par action de rayons UV obtenus au moyen d'une monosource lumineuse émettant des rayons de longueur d'onde 254 nm et des rayons à 185 nm et par photocatalyse le ratio quantité d'énergie des rayons à 185 nm/quantité d'énergie des rayons à 254 nm allant de 0,005 à 0,05;
2) le piégeage des polluants résiduels sur un support d'adsorption.

Dans un deuxième mode de réalisation particulier, le procédé (P2) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm, et par photocatalyse, le ratio quantité d'énergie des rayons à 185 nm/quantité d'énergie des rayons à 254 nm allant de 0,005 à 0,05 ;
2) la dégradation des polluants résiduels par une autre étape de photocatalyse.

Dans un troisième mode de réalisation particulier, le procédé (P3) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par une autre étape de photocatalyse ;
3) le piégeage des polluants résiduels sur un support d'adsorption.

Dans un quatrième mode de réalisation particulier, le procédé (P4) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par action de rayons UVC ;
3) éventuellement le piégeage des polluants résiduels sur un support d'adsorption.

Dans un cinquième mode de réalisation particulier, le procédé (P5) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par action de rayons UVA;
3) éventuellement le piégeage des polluants résiduels sur un support d'adsorption.

Dans un sixième mode de réalisation particulier, le procédé (P6) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par action de rayons UVC ;
3) la dégradation des polluants résiduels par action de rayons UVA ;
4) éventuellement le piégeage des polluants résiduels sur un support d'adsorption.

Dans un septième mode de réalisation (P7), une étape supplémentaire de photocatalyse peut être mise en oeuvre entre les étapes (2) et (3) des procédés (P4), (P5) et (P6).

Dans un huitième mode de réalisation particulier, le procédé (P8) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par une autre étape de photocatalyse ;
3) la dégradation des polluants résiduels par action de rayons UVC ;
4) éventuellement le piégeage des polluants résiduels sur un support d'adsorption, les étapes 2) et 3) pouvant être menées simultanément.

De préférence, les étapes 2) et 3) sont menées simultanément.

Dans un neuvième mode de réalisation particulier, le procédé (P9) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par une autre étape de photocatalyse ;
3) la dégradation des polluants résiduels par action de rayons UVA ;
4) éventuellement le piégeage des polluants résiduels sur un support d'adsorption, les étapes 2) et 3) pouvant être menées simultanément.

De préférence, les étapes 2) et 3) sont menées simultanément.

Dans un dixième mode de réalisation particulier, le procédé (P10) selon l'invention comprend les étapes suivantes :
1) la dégradation des polluants par ozonolyse, par action de rayons UV obtenus au moyen d'une monosource lumineuse émettant des rayons à 254 nm et des rayons à 185 nm et par photocatalyse, le ratio quantité d'énergie des rayons à 185 nm/quantité d'énergie des rayons à 254 nm allant de 0,005 à 0,05 ;
2) la dégradation des polluants résiduels par une autre étape de photocatalyse ;
3) la dégradation des polluants résiduels par action de rayons UVC ;
4) la dégradation des polluants résiduels par action de rayons UVA;
5) éventuellement le piégeage des polluants résiduels sur un support d'adsorption, les étapes 2), 3) et 4) pouvant être menées simultanément.

De préférence, les étapes 2), 3) et 4) sont menées simultanément.

Dans un onzième mode de réalisation (P11) une étape supplémentaire de photocatalyse peut être mise en oeuvre entre les étapes (3) et (4) des procédés (P8) et (P9) et entre les étapes (4) et (5) du procédé (P10).

Il doit être compris que dans les procédés (P1) à (P11) les rayons UVC et UVA agissant dans les étapes complémentaires 2, 3 et/ou 4 peuvent provenir d'au moins une autre source lumineuse ou de tout ou partie du quartz de la monosource émettant des rayons à 254 nm et le cas échéant des rayons UVA. La monosource émettant des rayons à 185 nm, 254 nm et des rayons UVA étant telle que définie plus haut.

De façon avantageuse, le procédé selon l'invention ainsi que le procédé selon chacun des modes de réalisation particuliers peut en outre comprendre une étape préalable de filtration mécanique de l'air permettant de retenir les impuretés les plus grosses en termes de taille. Cette filtration mécanique peut être réalisée par tout filtre mécanique connu de l'homme du métier, notamment à l'aide de filtre mécanique de type G (G1 à G4) définis selon la norme EN 779-2002, de préférence à l'aide d'un filtre G3 ; de type HEPA ou de type ULPA définis selon la norme EN 1822.

Le procédé de la présente invention permet avantageusement une purification de l'air très fine, la totalité ou la quasi-totalité des polluants étant dégradés ou piégés. De façon avantageuse, et contrairement au procédé de l'état de la technique, le procédé de l'invention permet également de dégrader les sous-produits issus de la dégradation des polluants. Ceci est notamment dû à la combinaison des différentes étapes du procédé selon l'invention.

De façon avantageuse, le procédé de l'invention permet de récupérer, après traitement un air dépollué répondant par exemple aux spécificités de la norme AFNOR XP B44-13 et comprenant pas ou peu d'ozone, de préférence entre 0,005 et 0,05 ppm d'ozone, par exemple entre 0,01 et 0,05 ppm d'ozone.

Le procédé de l'invention peut notamment être mis en oeuvre dans une application domestique du traitement de l'air, dans une application médicale du traitement de l'air, dans des applications du traitement de l'air très poussées. Le procédé de la présente invention peut ainsi et de façon avantageuse être mis en oeuvre pour le traitement d'air de salles d'hôpital, de salles blanches, de chambres froides, de salles d'élevage, de pièces domestiques, de pièces d'habitation, de bureaux, etc, et pour le traitement des rejets industriels.

Le procédé de l'invention peut également être avantageusement utilisé pour la désodorisation de l'air, notamment dans les applications précitées, par exemple pour le traitement des odeurs alimentaires, des rejets industriels, des odeurs de solvants, etc.

L'invention concerne également un dispositif pour la mise en oeuvre du procédé selon l'invention, tel que défini aux revendications.

De préférence, le ratio pour la monosource, quantité d'énergie des rayons à 185 nm/quantité d'énergie des rayons à 254 nm va de 0,005 à 0,05, pour une concentration en polluant allant de 1 à 5000 ppm, de préférence de 1 ppb à 100 ppm.

Le dispositif de la présente invention peut en outre comprendre dans la chambre (1) un support d'adsorption.

La monosource selon l'invention peut également émettre des rayons UVA émettant alors des rayons à 185 nm, 254 nm et des rayons UVA, le ratio quantité d'énergie des rayons à 185 nm/quantité d'énergie des rayons à 254 nm et des rayons UVA allant de 0,005 à 0,05 ; de préférence allant de 0,005 à 0,05 pour une concentration en polluant allant de 1 à 5000 ppm, de préférence allant de 1 ppb à 100 ppm.

Le dispositif de la présente invention peut en outre comprendre dans la chambre (1) une autre source lumineuse émettant des rayons UVA.

Il est divulgué que un dispositif comprenant en outre une chambre (2) comprenant :
- au moins une autre source lumineuse émettant des rayons UVC ; ou
- au moins une autre source lumineuse émettant des rayons UVA ; ou
- au moins une autre source lumineuse émettant des rayons UVC et au moins une autre source émettant des rayons UVA ; ou
- au moins une autre source lumineuse émettant des rayons UVC et un photocatalyseur ; ou
- au moins une autre source lumineuse émettant des rayons UVA et un photocatalyseur ; ou
- au moins une autre source lumineuse émettant des rayons UVC, au moins une autre source lumineuse émettant des rayons UVA et un photocatalyseur ; ou
- au moins une autre source lumineuse émettant des rayons UVC et un support d'adsorption ; ou
- au moins une autre source lumineuse émettant des rayons UVA et un support d'adsorption ; ou
- au moins une autre source lumineuse émettant des rayons UVC, au moins une autre source lumineuse émettant des rayons UVA et un support d'adsorption ; ou
- au moins une autre source lumineuse émettant des rayons UVC, un photocatalyseur et un support d'adsorption ; ou
- au moins une autre source lumineuse émettant des rayons UVA, un photocatalyseur et un support d'adsorption ; ou
- au moins une autre source lumineuse émettant des rayons UVA, au moins une autre source lumineuse émettant des rayons UVC, un photocatalyseur et un support d'adsorption ;
- un support d'adsorption.
- tout ou partie du quartz de la monosource émettant uniquement des rayons à 254 nm et le cas échéant uniquement des rayons à 254 nm et des rayons UVA, une autre source lumineuse émettant des rayons à 254 nm ou des rayons UVA, notamment des rayons UVA, et un photocatalyseur,

- tout ou partie du quartz de la monosource émettant uniquement des rayons à 254 nm et le cas échéant uniquement des rayons à 254 nm et des rayons UVA, une autre source lumineuse émettant des rayons à 254 nm ou des rayons UVA, notamment des rayons UVA, un photocatalyseur et un support d'adsorption.

Le dispositif de l'invention peut comprendre en outre une chambre (2) comprenant :
- tout ou partie du quartz de la monosource émettant uniquement des rayons à 254 nm et le cas échéant uniquement des rayons à 254 nm et des rayons UVA et un photocatalyseur,
- tout ou partie du quartz de la monosource émettant uniquement des rayons à 254 nm et le cas échéant uniquement des rayons à 254 nm et des rayons UVA, un photocatalyseur et un support d'adsorption,

De préférence, le dispositif de l'invention peut comprendre une chambre (2) comprenant :
- un photocatalyseur ; ou
- un support d'adsorption ; ou
- un photocatalyseur et un support d'adsorption, tout ou partie du quartz de la monosource émettant uniquement des rayons à 254 nm et le cas échéant uniquement des rayons à 254 nm et des rayons UVA nm .

La monosource étant telle que définie plus haut.

De préférence, la monosource présente un ratio quantité d'énergie des rayons à 185 nm/quantité d'énergie des rayons à 254 nm et rayons UVA) allant de 0,005 à 0,05 pour une concentration en polluant allant de 1 à 5000 ppm, de préférence de 1 à 100 ppm de préférence de 1 à 100 ppm, de manière plus préférée de 1 ppb à 10 ppm.

Dans un premier mode de réalisation (A), le dispositif selon l'invention comprend une chambre (1) comprenant une monosource lumineuse émettant des rayons à 254 nm et des rayons à 185 nm, un photocatalyseur, le ratio quantité d'énergie des rayons à 185 nm/quantité d'énergie des rayons à 254 nm allant de 0,005 à 0,05.

Dans un deuxième mode de réalisation (B), le dispositif selon l'invention comprend une chambre (1) comprenant une monosource lumineuse émettant des rayons à 254 nm et des rayons à 185 nm, un photocatalyseur et un support d'adsorption, le ratio quantité d'énergie des rayons à 185 nm/quantité d'énergie des rayons à 254 nm allant de 0,005 à 0,05.

Dans un troisième mode de réalisation (C), le dispositif selon l'invention comprend :
- une chambre (1) comprenant une monosource lumineuse émettant des rayons à 254 nm et des rayons à 185 nm et un photocatalyseur, le ratio quantité d'énergie des rayons à 185 nm/quantité d'énergie des rayons à 254 nm allant de 0,005 à 0,05 ;
- une chambre (2) comprenant au moins une autre source lumineuse émettant des rayons UVA et/ou UVC, et éventuellement un support d'adsorption ou comprenant tout ou partie du quartz de la monosource émettant uniquement des rayons à 254 nm et le cas échéant uniquement des rayons à 254 nm et des rayons UVA, et éventuellement un support d'adsorption.

Dans un quatrième mode de réalisation (D), le dispositif selon l'invention comprend :
- une chambre (1) comprenant une monosource lumineuse émettant des rayons à 254 nm et des rayons à 185 nm et un photocatalyseur, le ratio quantité d'énergie des rayons à 185 nm/quantité d'énergie des rayons à 254 nm allant de 0,005 à 0,05 ;
- une chambre (2) comprenant au moins une autre source lumineuse émettant des rayons UVA et/ou UVC, un photocatalyseur, et éventuellement un support d'adsorption ou comprenant tout ou partie du quartz de la monosource émettant uniquement des rayons à 254 nm et le cas échéant uniquement des rayons à 254 nm et des rayons UVA.

De préférence, il doit être compris, dans les dispositifs de l'invention comprenant plusieurs chambre et pour lesquels les chambres suivants la chambre 1 comprennent tout ou partie du quartz de la monosource émettant uniquement des rayons à 254 nm ou le cas échéant uniquement des rayons à 254 nm et des rayons UVA, que la monosource débute dans la première chambre dans laquelle se trouve le quartz émettant des rayons à 185 nm et des rayons à 254 nm ou le cas échéant des rayons à 185 nm, des rayons à 254 nm et des rayons UVA, et s'étend dans les autres chambres. La première chambre peut également comprendre une partie du quartz émettant uniquement des rayons à 254 nm ou le cas échéant uniquement des rayons à 254 nm et des rayons UVA.

Ces dispositifs particuliers permettent de préférence et de manière avantageuse de limiter la formation d'ozone dans la première chambre et par conséquent de limiter voire d'éliminer le relargage d'ozone à l'extérieur du dispositif.

Les dispositifs de la présente invention n'excluent pas la présence d'autres sources lumineuses, notamment émettant des rayons à 254 nm et des rayons UVA, dans les différentes chambres.

Dans les dispositifs de l'invention, la monosource peut également émettre des rayons UVA, la monosource émettant alors des rayons à 185 nm, 254 nm et des rayons UVA le ratio quantité d'énergie des rayons à 185 nm/quantité d'énergie des rayons à 254 nm et des rayons UVA allant de 0 0,005 à 0,05 ; de préférence allant de 0,005 à 0,05 pour une concentration en polluant allant de 1 à 5000 ppm, de préférence allant de 1 ppb à 100 ppm.

Pour ce qui concerne les dispositifs comprenant une seule chambre, les parois de cette chambre sont généralement perméables à l'air. Le photocatalyseur recouvre en tout ou partie la paroi interne de la chambre et le support d'adsorption, s'il est présent, recouvre en tout ou partie la paroi externe de la chambre.

Pour ce qui concerne les dispositifs comprenant au moins deux chambres, celles-ci sont juxtaposées les unes aux autres, la monosource émettant des rayons UV à 254 nm, à 185 nm et le cas échéant des rayons UVA est comprise dans la première chambre en contact avec l'air à traiter ; tout ou partie du quartz de la monosource émettant uniquement des rayons à 254 nm et le cas échéant uniquement des rayons à 254 nm et des rayons UVA peut s'étendre dans les autres chambres.

Cela permet, dans les autres chambres du dispositif de consommer l'ozone résiduel pour la dégradation des polluants ou par adsorption. Cela permet de façon avantageuse de limiter voire éviter le relargage d'ozone en fin de procédé.

Dans le cas où les chambres sont juxtaposées, seules les parois séparant les différentes chambres entre-elles sont perméables à l'air. Le photocatalyseur, s'il est présent peut recouvrir en tout ou partie les parois internes des chambres et/ou recouvrir en tout ou partie des plaques qui sont disposées à l'intérieur de la chambre de façon à ménager un passage pour l'air.

De façon avantageuse, les plaques s'étendent transversalement d'une paroi de la chambre en direction de la paroi opposée sans l'atteindre, de façon à ménager un espace entre la plaque et la paroi opposée permettant ainsi le passage de l'air. Ces plaques sont avantageusement disposées en alternance (une plaque en direction de la paroi inférieure et la plaque suivante en direction de la paroi supérieure). Cet arrangement particulier des plaques permet de forcer l'air à passer au contact des plaques ou a proximité. Cela permet une meilleure destruction des polluants par photocatalyse.

Dans un mode de réalisation préféré, le dispositif selon l'invention comprend deux chambres juxtaposées :
- une chambre (1) comprenant une monosource lumineuse émettant des rayons à 254 nm et 185 nm et un photocatalyseur, le ratio quantité d'énergie des rayons à 185 nm/quantité d'énergie des rayons à 254 nm allant de 0,005 à 0,05 ; et
- une chambre (2) comprenant tout ou partie du quartz de la monosource émettant uniquement des rayons à 254 nm, un photocatalyseur et éventuellement un support d'adsorption,

Seules les parois séparant les différentes chambres entre elles sont perméables à l'air. La chambre (2) peut éventuellement comprendre d'autres sources émettant des rayons à 254 nm.

De manière avantageuse, le dispositif selon l'invention comprend en outre un filtre mécanique de l'air.
La figure 1 représente une vue de face d'un dispositif (non conforme à l'invention) comprenant deux chambres cylindriques concentriques selon l'invention. La figure 1 est une vue de droite de la figure 2.
La figure 2 représente une coupe longitudinale d'un dispositif (non conforme à l'invention) comprenant deux chambres cylindriques concentriques selon l'invention.
La figure 3 représente un dispositif comprenant un assemblage de chambres juxtaposées selon l'invention.
La figure 4 représente une vue selon le plan de coupe IV d'un dispositif selon la figure 3.
La figure 5 représente une vue selon le plan de coupe IV d'un dispositif selon la figure 3.

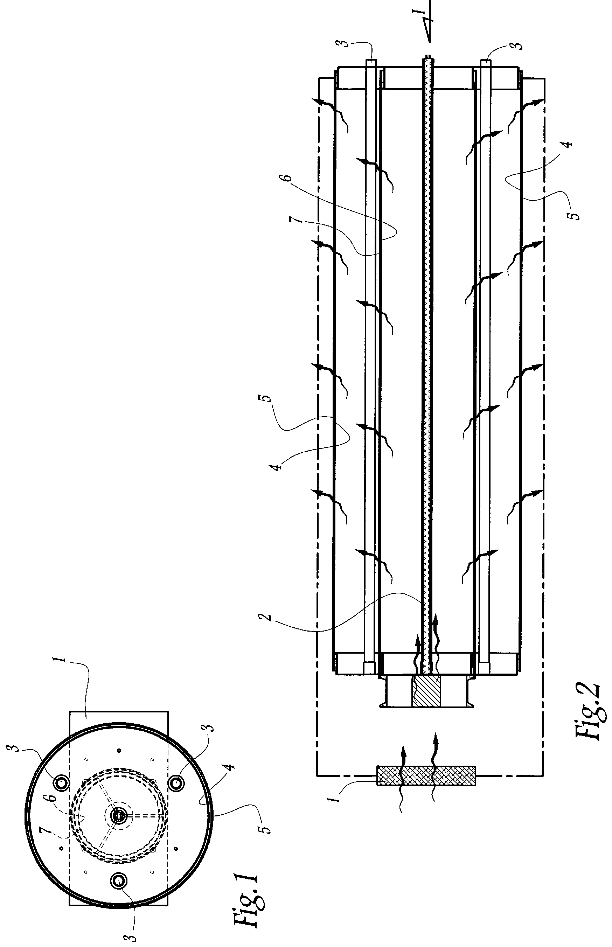

La figure 2 représente un dispositif (non conforme à l'invention) comprenant un filtre mécanique (1) et deux chambres cylindriques concentriques comprenant :
- une lampe monosource biquartz (2) émettant des rayonnements à 254 nm et des rayons à 185 nm situé dans la chambre interne ;
- trois lampes (3) (la troisième lampe étant dans le plan de la coupe) émettant des rayonnements à 254 nm et/ou à 365 nm situées dans la chambre externe ;
- un revêtement de photocatalyseur sur la paroi interne (4) de la chambre externe et un support d'adsorption sur la paroi externe (5) de la chambre externe ;
- un revêtement de photocatalyseur sur la paroi interne (6) et la paroi externe (7) de la chambre interne.

Les flux d'air sont matérialisés par les différentes flèches.

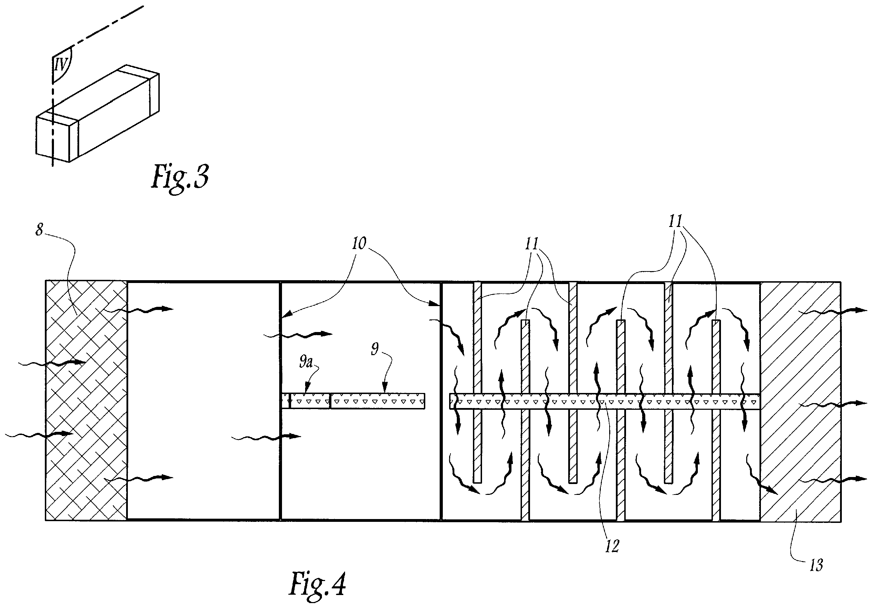

La figure 4 représente un dispositif selon l'invention comprenant un filtre mécanique (8), un support d'adsorption (13) et deux chambres juxtaposées :
- une première chambre comprenant une lampe biquartz (9) émettant des rayons à 254 nm et des rayons à 185 nm émis par la portion (9a), les parois internes (10) de cette chambre étant recouverte d'un photocatalyseur ; et
- une deuxième chambre comprenant une lampe émettant des rayons à 254 nm et/ou à 365 nm (12) et des plaques (11) dont la surface est recouverte d'un photocatalyseur.

Les flux d'air sont matérialisés par les différentes flèches. Le flux d'air vient au contact ou a proximité des plaques (11).

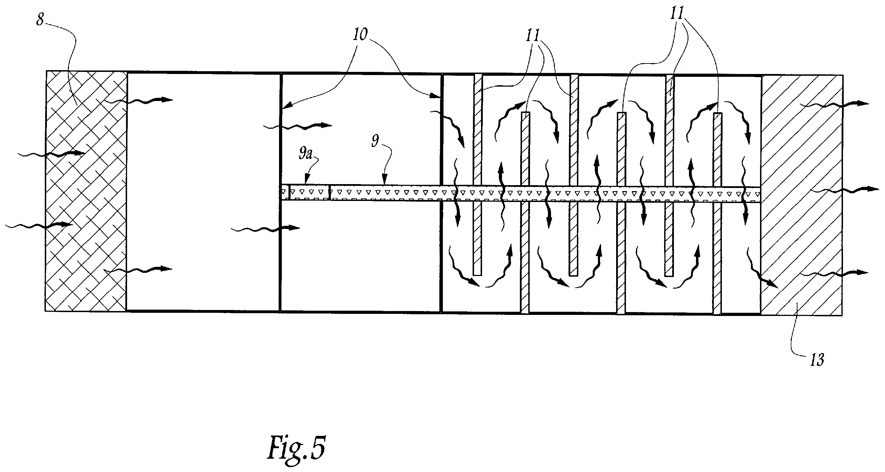

La figure 5 représente un dispositif selon l'invention comprenant un filtre mécanique (8), un support d'adsorption (13) et deux chambres juxtaposées :
- une première chambre comprenant une lampe biquartz (9) émettant des rayons à 254 nm et des rayons à 185 nm émis par la portion (9a), les parois internes (10) de cette chambre étant recouverte d'un photocatalyseur ; et
- une deuxième chambre comprenant des plaques (11) dont la surface est recouverte d'un photocatalyseur et dans laquelle s'étend la lampe (9) de la première chambre.

Les flux d'air sont matérialisés par les différentes flèches. Le flux d'air vient au contact ou a proximité des plaques (11).

La présente invention va maintenant être décrite à l'aide d'un exemple non limitatif.

### Exemple :

Le procédé selon l'invention a été mis en oeuvre pour traiter un air pollué comprenant 900 ppm d'éthylène et contenu dans une pièce de 54 m³.

Le dispositif de traitement consiste en deux chambres cylindriques concentriques (non conforme à l'invention) et un pré-filtre mécanique de type G3. La chambre interne comprend une lampe biquartz émettant des rayonnements à 254 nm et à 185 nm (1 à 2 % des rayons émis étant des rayons à 185 nm), la paroi interne et la paroi externe de cette chambre interne étant recouvertes de TiOs. La chambre externe comprend trois lampes émettant des rayonnements à 254 nm, la paroi interne de cette chambre étant recouverte de TiOs.

L'air passe dans le dispositif de traitement à un débit de 400 m³/heure.

Après 24 heures, la quantité d'éthylène dans l'air est de 135 ppm.

Cet exemple montre que ce procédé permet d'obtenir un air dépollué, et cela en un temps relativement court.

## Revendications

1. Procédé de purification d'air comprenant des polluants, comprenant la dégradation de tout ou partie des polluants par action de rayons UV obtenus au moyen d'un photocatalyseur et d'une monosource lumineuse bi-quartz, constituée de deux quartz, un premier quartz émettant des rayons à 254 nm et des rayons à 185 nm, les rayons à 185 nm produisant de l'ozone par réaction avec l'oxygène présent dans l'air à purifier, et un second quartz émettant des rayons uniquement à 254 nm, les rayons à 254 nm permettant la dégradation des polluants biologiques et une photocatalyse par le photocatalyseur, le premier quartz ayant une longueur et une surface inférieure au deuxième quartz et le ratio entre quantité d'énergie des rayons à 185 nm/quantité d'énergie des rayons à 254 nm allant de 0,005 à 0,05, le flux d'air à purifier passant d'abord au niveau du premier quartz, puis au niveau du deuxième quartz, ce grâce à quoi la production d'ozone est contrôlée.

2. Procédé selon la revendication 1 comprenant en outre la dégradation des polluants contenus dans l'air par l'action de rayons UVA émis au moyen de la monosource émettant alors des rayons à 185 nm, 254 nm et des rayons UVA, le ratio quantité d'énergie des rayons à 185 nm/quantité d'énergie des rayons à 254 nm et des rayons UVA allant de 0,005 à 0,05.

3. Procédé selon l'une des revendications 1 à 2 comprenant également:
- la dégradation des polluants par action de rayons UVA ; et/ou
- la dégradation des polluants par une autre étape de photocatalyse ; et/ou
- le piégeage des polluants par adsorption sur un support d'adsorption ; ou
- la dégradation des polluants par action de rayons UVC et par action de rayons UVA; ou
- la dégradation des polluants par action de rayons UVC, par action de rayons UVA, et la dégradation des polluants par une autre étape de photocatalyse ; ou
- la dégradation des polluants par action de rayons UVC, par action de rayons UVA, et le piégeage des polluants par adsorption sur un support d'adsorption ; ou
- la dégradation des polluants par action de rayons UVC, par action de rayons UVA, la dégradation des polluants par une autre étape de photocatalyse et le piégeage des polluants par adsorption sur un support d'adsorption ; ou
- la dégradation des polluants par action de rayons UVC et la dégradation des polluants par une autre étape de photocatalyse ; ou
- la dégradation des polluants par action de rayons UVC, la dégradation des polluants par une autre étape de photocatalyse et le piégeage des polluants par adsorption sur un support d'adsorption ; ou
- la dégradation des polluants par action de rayons UVC et le piégeage des polluants par adsorption sur un support d'adsorption ; ou
- la dégradation des polluants par action de rayons UVA, et la dégradation des polluants par une autre étape de photocatalyse ; ou
- la dégradation des polluants par action de rayons UVA, , la dégradation des polluants par une autre étape de photocatalyse et le piégeage des polluants par adsorption sur un support d'adsorption ; ou
- la dégradation des par action de rayons UVA, et le piégeage des polluants par adsorption sur un support d'adsorption ; ou
- la dégradation des polluants par une autre étape de photocatalyse et le piégeage des polluants par adsorption sur un support d'adsorption.
les rayons UVC et UVA provenant de tout ou partie du quartz de la monosource émettant uniquement des rayons à 254 nm ou le cas échéant uniquement des rayons à 254 nm et des rayons UVA.

4. Procédé selon l'une des revendications 1 à 3, comprenant en outre le piégeage des polluants résiduels sur un support d'adsorption ou la dégradation des polluants résiduels par une autre étape de photocatalyse.

5. Procédé selon l'une des revendications 1 à 4, pour lequel la photocatalyse est mise en oeuvre en présence d'un catalyseur choisi parmi les photocatalyseurs supportés ou non supportés et comprenant du titane ou du tungstène.

6. Procédé selon la revendication 5, pour lequel le photocatalyseur est supporté sur du papier ; de la silice ou des composés comprenant de la silice, par exemple le quartz ; des tissus, notamment tissus de verre, par exemple tissus de quartz et silice ; des fibres de verre ; des tissus de fibres de verre ; des tissus de fibres optiques ; des céramiques ; ou un assemblage de ces supports.

7. Procédé selon la revendication 5 ou 6, pour lequel le photocatalyseur est du TiOs supporté.

8. Procédé selon l'une des revendications 3 à 7, pour lequel le support d'adsorption est choisi parmi du charbon actif, des zéolithes, des xérogels de silice, ou leurs mélanges.

9. Procédé selon l'une des revendications 1 à 8, comprenant une étape préalable de filtration mécanique de l'air.

10. Dispositif adapté à la mise en oeuvre du procédé selon les revendications 1 à 9, comprenant une chambre (1) comprenant une monosource lumineuse bi-quartz (2, 9), constituée de deux quartz, un premier quartz (9a) émettant des rayons à 254 nm et à 185 nm et un second quartz émettant des rayons uniquement à 254 nm, et un photocatalyseur, le premier quartz présentant une longueur et une surface inférieure au deuxième quartz, le ratio quantité d'énergie des rayons à 185 nm/quantité d'énergie des rayons à 254 nm allant de 0,005 à 0,05, le deuxième quartz s'étendant dans le dispositif après le premier quartz par rapport à la direction de circulation de l'air à purifier.

11. Dispositif selon la revendication 10, pour lequel la monosource émet en outre des rayons UVA, la monosource émettant alors des rayons à 185 nm, 254 nm et des rayons UVA le ratio quantité d'énergie des rayons à 185 nm/quantité d'énergie des rayons à 254 nm et des rayons UVA allant de 0,005 à 0,05.

12. Dispositif selon la revendication 10 ou 11, dont la chambre (1) comprend également un support d'adsorption.

13. Dispositif selon l'une des revendications 10 à 12, comprenant en outre une deuxième chambre (2) comprenant :
- tout ou partie du quartz de la monosource émettant uniquement des rayons à 254 nm et le cas échéant uniquement des rayons à 254 nm et des rayons UVA et un photocatalyseur,
- tout ou partie du quartz de la monosource émettant uniquement des rayons à 254 nm et le cas échéant uniquement des rayons à 254 nm et des rayons UVA, un photocatalyseur et un support d'adsorption.

14. Dispositif selon l'une des revendications 10 à 13, choisi parmi :
- dispositif (A), comprenant une chambre (1) comprenant une monosource lumineuse émettant des rayons à 254 nm et des rayons à 185 nm et un photocatalyseur, le ratio quantité d'énergie des rayons à 185 nm/quantité d'énergie des rayons à 254 nm allant de 0,005 à 0,05 ;
- dispositif (B) comprenant une chambre (1) comprenant une monosource lumineuse émettant des rayons à 254 nm et des rayons à 185 nm, un photocatalyseur et un support d'adsorption, le ratio quantité d'énergie des rayons à 185 nm/quantité d'énergie des rayons à 254 nm allant de 0,005 à 0,05 ;
- dispositif (C) comprenant :
- une première chambre (1) comprenant une monosource lumineuse émettant des rayons à 254 nm et des rayons à 185 nm et un photocatalyseur, le ratio quantité d'énergie des rayons à 185 nm/quantité d'énergie des rayons à 254 nm allant de 0,005 à 0,05 ;
- une deuxième chambre (2) comprenant tout ou partie du quartz de la monosource émettant uniquement des rayons à 254 nm et le cas échéant uniquement des rayons à 254 nm et des rayons UVA, et éventuellement un support d'adsorption.
- dispositif (D), le dispositif comprenant :
- une première chambre (1) comprenant une monosource lumineuse émettant des rayons à 254 nm et des rayons à 185 nm et un photocatalyseur, le ratio quantité d'énergie des rayons à 185 nm/quantité d'énergie des rayons à 254 nm allant de 0,005 à 0,05 ;
- une deuxième chambre (2) comprenant tout ou partie du quartz de la monosource émettant uniquement des rayons à 254 nm et le cas échéant uniquement des rayons à 254 nm et des rayons UVA.

15. Dispositif selon la revendication 10, comprenant deux chambres juxtaposées :
- une première chambre (1) comprenant une monosource lumineuse émettant des rayons à 254 nm et 185 nm et un photocatalyseur, le ratio quantité d'énergie des rayons à 185 nm/quantité d'énergie des rayons à 254 nm allant de 0,005 à 0,05 ; et
- une deuxième chambre (2) comprenant tout ou partie du quartz de la monosource émettant uniquement des rayons à 254 nm, un photocatalyseur et éventuellement un support d'adsorption,
seules les parois séparant les différentes chambres entre elles sont perméables à l'air, le photocatalyseur recouvre en tout ou partie les parois internes des chambres (1, 2) et/ou des plaques qui sont disposées à l'intérieur de la chambre de façon à ménager un passage pour l'air.

16. Dispositif selon l'une quelconque des revendications 10 à 15, comprenant en outre un filtre mécanique de l'air.

17. Dispositif selon la revendication 10, **caractérisé en ce qu'**il comprend une seule chambre (1) et le photocatalyseur recouvre en tout ou partie la paroi interne de la chambre.

## Patentansprüche

1. Verfahren zur Reinigung von Luft, die Schadstoffe enthält, umfassend den Abbau aller oder eines Teils der Schadstoffe durch die Einwirkung von UV-Strahlen, die mittels eines Photokatalysators und einer Bi-Quarz-Monolichtquelle erhalten werden, die aus zwei Quarzen besteht, wobei ein erster Quarz Strahlen bei 254 nm und Strahlen bei 185 nm emittiert. Die Strahlen bei 185 nm erzeugen Ozon durch Reaktion mit dem Sauerstoff in der zu reinigenden Luft, und ein zweiter Quarz emittiert nur Strahlen bei 254 nm, wobei die Strahlen bei 254 nm den Abbau biologischer Schadstoffe und eine Photokatalyse durch den Photokatalysator ermöglichen, wobei der erste Quarz eine geringere Länge und Oberfläche als der zweite Quarz aufweist und das Verhältnis zwischen Energiemenge der Strahlen bei 185 nm und Energiemenge der Strahlen bei 254 nm von 0,005 bis 0,05 beträgt, wobei der zu reinigende Luftstrom zuerst den ersten Quarz und dann den zweiten Quarz passiert, wodurch die Ozonproduktion gesteuert wird.

2. Verfahren nach Anspruch 1, das weiterhin den Abbau von in der Luft enthaltenen Schadstoffen durch die Wirkung von UVA-Strahlen umfasst, die mittels der Monoquelle emittiert werden, die dann Strahlen bei 185 nm, 254 nm und UVA-Strahlen emittiert, wobei das Verhältnis Energiemenge der Strahlen bei 185 nm und Energiemenge der Strahlen bei 254 nm und der UVA-Strahlen 0,005 bis 0,05 beträgt.

3. Verfahren nach einem der Ansprüche 1 bis 2, auch umfassend:
- den Abbau der Schadstoffe durch Einwirkung von UVA-Strahlen; und/oder
- den Abbau der Schadstoffe durch einen weiteren Schritt der Photokatalyse; und/oder
- das Einfangen der Schadstoffe durch Adsorption an einem Adsorptionsträger; oder
- den Abbau der Schadstoffe durch Einwirkung von UVC- und UVA-Strahlen; oder
- den Abbau der Schadstoffe durch Einwirkung von UVC-Strahlen, durch Einwirkung von UVA-Strahlen und den Abbau der Schadstoffe durch einen weiteren Schritt der Photokatalyse; oder
- den Abbau der Schadstoffe durch Einwirkung von UVC-Strahlen, durch Einwirkung von UVA-Strahlen und das Einfangen der Schadstoffe durch Adsorption an einem Adsorptionsträger; oder
- den Abbau der Schadstoffe durch Einwirkung von UVC-Strahlen, durch Einwirkung von UVA-Strahlen, den Abbau der Schadstoffe durch einen weiteren Schritt der Photokatalyse und das Einfangen der Schadstoffe durch Adsorption an einem Adsorptionsträger; oder
- den Abbau der Schadstoffe durch Einwirkung von UVC-Strahlen und den Abbau der Schadstoffe durch einen weiteren Schritt der Photokatalyse; oder
- den Abbau der Schadstoffe durch Einwirkung von UVC-Strahlen, den Abbau der Schadstoffe durch einen weiteren Schritt der Photokatalyse und das Einfangen der Schadstoffe durch Adsorption an einem Adsorptionsträger; oder
- den Abbau der Schadstoffe durch die Einwirkung von UVC-Strahlen und das Einfangen der Schadstoffe durch Adsorption an einem Adsorptionsträger; oder
- den Abbau der Schadstoffe durch Einwirkung von UVA-Strahlen und den Abbau der Schadstoffe durch einen weiteren Schritt der Photokatalyse; oder
- den Abbau der Schadstoffe durch Einwirkung von UVA-Strahlen, den Abbau der Schadstoffe durch einen weiteren Schritt der Photokatalyse und das Einfangen der Schadstoffe durch Adsorption auf einem Adsorptionsträger; oder
- den Abbau der Schadstoffe durch Einwirkung von UVA-Strahlen und das Einfangen der Schadstoffe durch Adsorption an einem Adsorptionsträger; oder
- den Abbau der Schadstoffe durch einen weiteren Schritt der Photokatalyse und das Einfangen der Schadstoffe durch Adsorption an einem Adsorptionsträger.
UVC- und UVA-Strahlung aus dem gesamten oder einem Teil des Quarzes der Monoquelle, die nur 254 nm-Strahlung oder gegebenenfalls nur 254 nm- und UVA-Strahlung aussendet.

4. Verfahren nach einem der Ansprüche 1 bis 3, das außerdem das Einfangen der restlichen Schadstoffe auf einem Adsorptionsträger oder den Abbau der restlichen Schadstoffe durch einen weiteren Schritt der Photokatalyse umfasst.

5. Verfahren nach einem der Ansprüche 1 bis 4, bei dem die Photokatalyse in Gegenwart eines Katalysators durchgeführt wird, der aus geträgerten oder ungeträgerten Photokatalysatoren ausgewählt ist und Titan oder Wolfram umfasst.

6. Verfahren nach Anspruch 5, bei dem der Photokatalysator auf Papier; Siliciumdioxid oder Siliciumdioxid enthaltende Verbindungen, z.B. Quarz; Gewebe, insbesondere Glasgewebe, z.B. Quarz- und Siliciumgewebe; Glasfasern; Glasfasergewebe; faseroptische Gewebe; Keramiken; oder einer Anordnung dieser Träger geträgert wird.

7. Verfahren nach Anspruch 5 oder 6, bei dem der Photokatalysator geträgertes TiO₂ ist.

8. Verfahren nach einem der Ansprüche 3 bis 7, bei dem der Adsorptionsträger aus Aktivkohle, Zeolithen, Siliciumdioxid-Xerogelen oder Gemischen davon ausgewählt ist.

9. Verfahren nach einem der Ansprüche 1 bis 8, das einen vorherigen Schritt der mechanischen Filterung der Luft umfasst.

10. Vorrichtung, die zur Durchführung des Verfahrens nach den Ansprüchen 1 bis 9 geeignet ist, mit einer Kammer (1), die eine Bi-Quarz-Monolichtquelle (2, 9) enthält, bestehend aus zwei Quarzen, wobei ein erster Quarz (9a) Strahlen bei 254 nm und 185 nm und ein zweiter Quarz nur Strahlen bei 254 nm emittiert, und einem Photokatalysator, wobei der erste Quarz eine geringere Länge und Oberfläche als der zweite Quarz aufweist, wobei das Verhältnis der Energiemenge der 185 nm-Strahlen zur Energiemenge der 254 nm-Strahlen von 0,005 bis 0,05 beträgt, wobei sich der zweite Quarz in der Vorrichtung nach dem ersten Quarz in Bezug auf die Strömungsrichtung der zu reinigenden Luft erstreckt.

11. Vorrichtung nach Anspruch 10, bei der die Monoquelle zusätzlich UVA-Strahlen emittiert, wobei die Monoquelle dann Strahlen bei 185 nm, 254 nm und UVA-Strahlen emittiert, wobei das Verhältnis Energiemenge der Strahlen bei 185 nm zur Energiemenge der Strahlen bei 254 nm und der UVA-Strahlen von 0,005 bis 0,05 reicht.

12. Vorrichtung nach Anspruch 10 oder 11, deren Kammer (1) auch einen Adsorptionsträger umfasst.

13. Vorrichtung nach einem der Ansprüche 10 bis 12, die außerdem eine zweite Kammer (2) umfasst, die:
- den gesamten oder einen Teil des Quarzes der Monoquelle, die nur Strahlung bei 254 nm und gegebenenfalls nur Strahlung bei 254 nm und UVA-Strahlung aussendet, und einen Photokatalysator umfasst,
- den gesamten oder einen Teil des Quarzes der Monoquelle, die nur Strahlung bei 254 nm und gegebenenfalls nur Strahlung bei 254 nm und UVA-Strahlung emittiert, einen Photokatalysator und einen Adsorptionsträger.

14. Vorrichtung nach einem der Ansprüche 10 bis 13, ausgewählt aus Folgendem:
- Vorrichtung (A), umfassend eine Kammer (1), die eine Monolichtquelle, die Strahlen bei 254 nm und Strahlen bei 185 nm emittiert, und einen Photokatalysator umfasst, wobei das Verhältnis der Energiemenge der Strahlen bei 185 nm zur Energiemenge der Strahlen bei 254 nm von 0,005 bis 0,05 reicht;
- Vorrichtung (B) mit einer Kammer (1), die eine Monolichtquelle, die Strahlen bei 254 nm und Strahlen bei 185 nm emittiert, einen Photokatalysator und einen Adsorptionsträger umfasst, wobei das Verhältnis Energiemenge der Strahlen bei 185 nm zur Energiemenge der Strahlen bei 254 nm von 0,005 bis 0,05 reicht;
- Vorrichtung (C), umfassend:
- eine erste Kammer (1) mit einer Monolichtquelle, die Strahlen bei 254 nm und Strahlen bei 185 nm emittiert, und einem Photokatalysator, wobei das Verhältnis der Energiemenge der Strahlen bei 185 nm zur Energiemenge der Strahlen bei 254 nm von 0,005 bis 0,05 reicht;
- eine zweite Kammer (2), die den gesamten oder einen Teil des Quarzes der Monoquelle, die nur 254 nm-Strahlung und gegebenenfalls nur 254 nm-Strahlung und UVA-Strahlung emittiert, und gegebenenfalls einen Adsorptionsträger umfasst.
- Vorrichtung (D), wobei die Vorrichtung umfasst:
- eine erste Kammer (1) mit einer Monolichtquelle, die Strahlen bei 254 nm und Strahlen bei 185 nm emittiert, und einem Photokatalysator, wobei das Verhältnis der Energiemenge der Strahlen bei 185 nm zur Energiemenge der Strahlen bei 254 nm von 0,005 bis 0,05 reicht;
- eine zweite Kammer (2), die den gesamten oder einen Teil des Quarzes der Monoquelle umfasst, die nur 254 nm-Strahlen und gegebenenfalls nur 254 nm-Strahlen und UVA-Strahlen emittiert.

15. Vorrichtung nach Anspruch 10 mit zwei nebeneinander liegenden Kammern:
- eine erste Kammer (1) mit einer Monolichtquelle, die Strahlen bei 254 nm und 185 nm emittiert, und einem Photokatalysator, wobei das Verhältnis Energiemenge der Strahlen bei 185 nm zur Energiemenge der Strahlen bei 254 nm von 0,005 bis 0,05 reicht; und
- eine zweite Kammer (2), die den gesamten oder einen Teil des Quarzes der Monoquelle, die nur Strahlen bei 254 nm aussendet, einen Photokatalysator und gegebenenfalls einen Adsorptionsträger umfasst, wobei nur die Wände, die die verschiedenen Kammern voneinander trennen, luftdurchlässig sind. Der Photokatalysator bedeckt ganz oder teilweise die Innenwände der Kammern (1, 2) und/oder Platten, die im Inneren der Kammer angeordnet sind, um einen Durchgang für die Luft zu schaffen.

16. Vorrichtung nach einem der vorherigen Ansprüche 10 bis 15, ferner umfassend einen mechanischen Luftfilter.

17. Vorrichtung nach Anspruch 10, **dadurch gekennzeichnet, dass** sie eine einzelne Kammer (1) umfasst und der Photokatalysator die Innenwand der Kammer ganz oder teilweise bedeckt.

## Claims

1. A method for purifying air comprising pollutants, comprising the degradation of all or part of the pollutants by the action of UV rays obtained by means of a photocatalyst and a single biquartz light source, made up of two quartz, a first quartz emitting rays at 254 nm and rays at 185 nm, rays at 185 nm producing ozone by reaction with the oxygen present in the air to be purified, and a second quartz emitting rays only at 254 nm, the 254 nm rays allowing biological pollutants to be degraded, and by photocatalysis by the photocatalyst, the first quartz having a length and surface less than the second quartz and the ratio between the amount of energy of the rays at 185 nm and the amount of energy of the rays at 254 nm ranging from 0.005 to 0.05, with the air flow to be purified passing first through the first quartz and then through the second quartz, thereby controlling ozone production.

2. The method according to claim 1, further comprising the degradation of the pollutants contained in the air by the action of UVA rays emitted by means of the single source then emitting rays at 185 nm, 254 nm and UVA rays, the ratio between the amount of energy of the rays at 185 nm and the amount of energy of the rays at 254 nm and of the UVA rays ranging from 0.005 to 0.05.

3. The method according to one of claims 1 to 2 also comprising:
- degradation of the pollutants by the action of UVA rays; or
- degradation of the pollutants with another photocatalysis step; and/or
- scavenging the pollutants by adsorption on an adsorption supporting medium; or
- degradation of the pollutants by the action of UVC rays and by the action of UVA rays; or
- degradation of the pollutants by the action of UVC rays, by the action of UVA rays and degradation of the pollutants with another photocatalysis step; or
- degradation of the pollutants by the action of UVC rays, by reaction of UVA rays and the scavenging of the pollutants by adsorption on an adsorption supporting medium; or
- degradation of the pollutants by the action of UVC rays, by the action of UVA rays, the degradation of the pollutants with another photocatalysis step and scavenging of the pollutants by adsorption on an adsorption supporting medium; or
- degradation of the pollutants by the action of UVC rays and degradation of the pollutants with another photocatalysis step; or
- degradation of the pollutants by the action of UVC rays, degradation of the pollutants with another photocatalysis step and scavenging of the pollutants by adsorption on an adsorption supporting medium; or
- degradation of the pollutants by the action of UVC rays and scavenging of the pollutants by adsorption on an adsorption supporting medium; or
- degradation of the pollutants by the action of UVA rays, and degradation of the pollutants with another photocatalysis step; or
- degradation of the pollutants by the action of UVA rays, and degradation of the pollutants with another photocatalysis step and scavenging of the pollutants by adsorption on an adsorption supporting medium; or
- degradation of the pollutants by the action of UVA rays, and scavenging of the pollutants by adsorption on an adsorption supporting medium; or
- degradation of the pollutants with another photocatalysis step and scavenging of the pollutants by adsorption on an adsorption supporting medium,
the UVC and UVA rays stemming from all or part of the quartz of the single source only emitting rays at 254 nm or if necessary only rays at 254 nm and UVA rays.

4. The method according to one of claims 1 to 3, further comprising scavenging of the residual pollutants on an adsorption supporting medium or degradation of the residual pollutants with another photocatalysis step.

5. The method according to one of claims 1 to 4, for which the photocatalysis is applied in the presence of a catalyst selected from supported or unsupported photocatalysts and comprising titanium or tungsten.

6. The method according to claim 5, for which the photocatalyst is supported on paper; on silica or compounds comprising silica, for example quartz; on fabrics, notably glass fabrics, for example quartz and silica fabrics; on glass fibers; on glass fiber fabrics; on optical fiber fabrics; on ceramics; or on an assembly of these supports

7. The method according to claim 5 or 6, for which the photocatalyst is supported TiO₂.

8. The method according to one of claims 3 to 7, for which the adsorption supporting medium is selected from active coal, zeolites, silica xerogels, or mixtures thereof.

9. The method according to one of claims 1 to 8, comprising a preliminary step for mechanical filtering of air.

10. A device adapted for applying the method according to claims 1 to 9, comprising a chamber (1) comprising a single biquartz light source (2, 9) made up of two quartz, a first quartz (9a) emitting rays at 254 nm and at 185 nm and a second quartz emitting rays only at 254 nm, and a photocatalyst, the first quartz having a length and surface less than the second quartz, the ratio between the amount of energy of the rays at 185 nm and the amount of energy of the rays at 254 nm ranging from 0.005 to 0.05, the second quartz extending in the device after the first quartz with respect to the direction of flow of the air to be purified.

11. The device according to claim 10, for which the single source further emits UVA rays, the single source then emitting rays at 185 nm, 254 nm and UVA rays, the ratio between the amount of energy of the rays at 185 nm /amount of energy of the rays at 254 nm and of the UVA rays ranging from 0.005 to 0.05.

12. The device according to claim 10 or 11, the chamber (1) of which also comprises an adsorption supporting medium.

13. The device according to one of claims 10 or 12, further comprising a second chamber (2) comprising:
- all or part of the quartz of the single source only emitting rays at 254 nm and if necessary only rays at 254 nm and UVA rays and a photocatalyst;
- all or part of the quartz of the single source only emitting rays at 254 nm and if necessary only rays at 254 nm and UVA rays, a photocatalyst and an adsorption supporting medium.

14. The device according to one of claims 10 to 13, selected from:
- a device (A) comprising a chamber (1) comprising a single light source emitting rays at 254 nm and rays at 185 nm and a photocatalyst, the ratio between the amount of energy of the rays at 185 nm and the amount of energy of the rays at 254 nm ranging from 0.005 to 0.05;
- a device (B) comprising a chamber (1) comprising a single light source emitting rays at 254 nm and rays at 185 nm a photocatalyst and an adsorption supporting medium, the ratio between the amount of energy of the rays at 185 nm and the amount of energy of the rays at 254 nm ranging from 0.005 to 0.05;
- a device (C) comprising:
- a first chamber (1) comprising a single light source emitting rays at 254 nm and rays at 185 nm and a photocatalyst, the ratio between the amount of energy of the rays at 185 nm and the amount of energy of the rays at 254 nm ranging from 0.005 to 0.05;
- a second chamber (2) comprising all or part of the quartz of the single source only emitting rays at 254 nm and if necessary only rays at 254 nm and UVA rays, and optionally an adsorption supporting medium,
- a device (D), the device comprising:
- a first chamber (1) comprising a single light source emitting rays at 254 nm and rays at 185 nm and a photocatalyst, the ratio between the amount of energy of the rays at 185 nm and the amount of energy of the rays at 254 nm ranging from 0.005 to 0.05;
- a second chamber (2) comprising all or part of the quartz of the single source only emitting rays at 254 nm and if necessary only rays at 254 nm and UVA rays.

15. The device according to claim 10, comprising two juxtaposed chambers:
- a first chamber (1) comprising a single light source emitting rays at 254 nm and 185 nm and a photocatalyst, the ratio between the amount of energy of the rays at 185 nm and the amount of energy of the rays at 254 nm ranging from 0.005 to 0.05;
- a second chamber (2) comprising all or part of the quartz of the single source only emitting rays at 254 nm, a photocatalyst and optionally an adsorption supporting medium,
only the walls separating the different chambers from each other are permeable to air, the photocatalyst covers all or part of the internal walls of the chambers (1, 2) and/or of the plates which are positionned inside the chamber so as to provide a passage for the air.

16. The device according to any of claims 10 to 15, further comprising a mechanical air filter.

17. The device according to claim 10, **characterised in that** it comprises a single chamber (1) and the photocatalyst covers all or part of the internal wall of the chamber.
